(19) Europäisches Patentamt
European Patent Office
Office européen des brevets

(11) Veröffentlichungsnummer: **0 007 572**
**B1**

(12) # EUROPÄISCHE PATENTSCHRIFT

(45) Veröffentlichungstag der Patentschrift: **27.04.83**

(51) Int. Cl.³: **A 61 K 45/06** // (A61K45/06, 31/13),(A61K45/06, 31/16),(A61K45/06, 31/40)

(21) Anmeldenummer: **79102529.9**

(22) Anmeldetag: **18.07.79**

(54) **Pharmazeutische Präparate mit einem Gehalt an einer Amantadin-Verbindung.**

(30) Priorität: **21.07.78 CH 7905/78**

(43) Veröffentlichungstag der Anmeldung: **06.02.80 Patentblatt 80/3**

(45) Bekanntmachung des Hinweises auf die Patenterteilung: **27.04.83 Patentblatt 83/17**

(84) Benannte Vertragsstaaten: **DE GB**

(56) Entgegenhaltungen:
**CITET DOCUMENTS (56):**
**Rote Liste 1977/78, Nr. 77037B**
**B. Helwig: Moderne Arzneimittel, Stuttgart 1980, S. 574**
**UNLISTED DRUGS, vol. 25, Nr. 2, Februar 1973, 193p. Chatham, N.Y. (USA) «Nostress»**
**UNLISTED DRUGS, vol. 25, Nr. 2, Februar 1973, 21d. Chatham, N.Y. (USA) «Bellosin»**
**CHEMICAL ABSTRACTS, Band 68, Nr. 3, 15. Januar 1968, Seite 1186, Spalte 1, Zusammenfassung 12560z. Columbus, Ohio, USA**
**CHEMICAL ABSTRACTS, Band 69, Nr. 17, 21. Oktober 1968, Seite 6248, Spalte 1, Zusammenfassung 66989k. Columbus, Ohio, USA**

(73) Patentinhaber: **CIBA-GEIGY AG, Patentabteilung Postfach, CH-4002 Basel (CH)**

(72) Erfinder: **Kradolfer, Friedrich, Dr., Bruderholzallee 202, CH-4059 Basel (CH)**
Erfinder: **Lukas, Bohumir, Dr., Laufenburgerstrasse 10/3, CH-4058 Basel (CH)**
Erfinder: **Schmidt-Ruppin, Karl Heinz, Dr., Im Baumgarten 5, CH-4144 Arlesheim (CH)**

(74) Vertreter: **Zumstein, Fritz sen., Dr. et al, Bräuhausstrasse 4, D-8000 München 2 (DE)**

(56) **Die Akte enthält technische Angaben, die nach dem Eingang der Anmeldung eingereicht wurden und die nicht in dieser Patentschrift enthalten sind.**

**EP 0 007 572 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Pharmazeutische Präparate mit einem Gehalt an einer Amantadinverbindung

Die Erfindung betrifft neue synergistisch wirksame pharmazeutische Präparate zur Behandlung von Virusinfektionen enthaltend eine antiviral wirksame Verbindung vom Amantadin-Typ in Kombination mit Salicylamid oder Propyphenazon sowie gegebenenfalls bestimmten weiteren pharmakologischen Wirkstoffen.

Es ist bekannt, dass antiviral-wirksame Verbindungen vom Amantadin-Typ, insbesondere Amantadin selber, d.h. 1-Amino-adamantan, oder ein pharmazeutisch annehmbares, nicht-toxisches Säureadditionssalz davon, insbesondere das Hydrochlorid, gegen Influenza-Viren, in erster Linie gegen Influenza A-Viren und insbesondere gegen Influenza A($H_3N_2$)-Viren, wie z.B. Influenza A Hongkong 1/68, Influenza A England 42/72, Influenza A Port Chalmers 1/73 und Influenza A Victoria 3/75, wirksam sind. Eine ausgeprägte antivirale Wirkung kommt jedoch nur bei prophylaktischer Verabreichung, d.h. bei der Behandlung vor dem Ausbruch der eigentlichen Erkrankung oder im frühesten Stadium des Erkrankungsbeginnes zustande. Im späteren Krankheitsstadium kann sich, bei weitgehend abgeschlossener Virusreplikation, der in der Hemmung der Viruspenetration durch die Zellmembran bestehende antivirale Effekt nicht mehr auswirken. Die bei hoher Dosierung von über 200 mg/Tag beobachteten Nebenwirkungen der Verbindungen vom Amantadin-Typ, insbesondere auf das Zentralnervensystem, und der medizinische und wirtschaftliche Aufwand limitieren jedoch ihre prophylaktische praktische Anwendung. Den anërkanntermassen guten antiviralen Eigenschaften der Verbindungen vom Amantadin-Typ steht somit eine beschränkte Verwendungsmöglichkeit gegenüber, die bisher eine breite Allgemeinverwendung dieser Verbindungen bei Virus-, insbesondere Influenzavirus-Erkrankungen, verhindert hat.

Es wurde nun überraschenderweise gefunden, dass antiviral wirksame Verbindungen vom Amantadin-Typ bei Infektionen mit Influenza-Viren bei relativ niedriger Dosierung therapeutisch, d.h. nach erfolgter Virusinfektionen, verwendet werden können, wenn man sie zusammen mit Salicylamid oder Propylphenazon (chemische Bezeichnung 2,3-Dimethyl-3-isopropyl-1-phenyl-3-pyrazolin-5-on) verabreicht. So konnte z.B. anhand von Tierversuchen, wie etwa an Mäusen, gezeigt werden, dass bei der gemeinsamen Verabreichung der antiviral und der antiphlogistischantipyretisch-analgetisch-wirksamen Verbindungen ab 60 Stunden nach erfolgter Infektion mit einer Dosis eines Influenzavirus, wie eines der obgenannten, z.B. Influenza A Port Chalmers 1/73 oder Influenza A/Victoria 3/75 ($H_3N_2$), die für 90% oder ca. 50% der damit behandelten Versuchstiere tödlich ist ($LD_{90}$ bzw. $LD_{50}$), die Überlebensrate der Versuchstiere signifikant erhöht und der durch die Infektion bedingte Gewichtsverlust vermieden werden kann.

Ein zusätzlicher Effekt kann noch festgestellt werden, wenn man zusammen mit einer antiviral wirksamen Verbindung vom Amantadin-Typ und Salicylamid oder Propyphenazon auch noch eine antiallergisch wirksame und/oder eine auf α-adrenergische Rezeptoren blockierend wirkende Verbindung verabreicht.

Die Erfindung betrifft deshalb synergistisch wirksame, pharmazeutische Präparate zur Behandlung von Virusinfektionen die eine antiviral wirksame Verbindung vom Amantadin-Typ oder ein pharmazeutisch annehmbares Säureadditionssalz einer solchen in Kombination mit Salicylamid oder Propyphenazon und gewünschtenfalls zusätzlich eine antiallergisch wirksame Verbindung und/oder eine auf α-adrenergische Rezeptoren blockierend wirkende Verbindung enthalten, und zur Behandlung von Viruserkrankungen, insbesondere von Influenzaviruserkrankungen verwendet werden können.

In den erfindungsgemässen pharmazeutischen Präparaten kann die antiviral wirksame Verbindung vom Amantadin-Typ, gegebenenfalls in Form eines pharmazeutisch annehmbaren, nicht-toxischen Säureadditionssalzes vorliegen, ebenso können die gegebenenfalls vorhandenen weiteren pharmakologisch wirksamen Stoffe je nach ihrer Struktur auch als solche Salze oder als pharmazeutisch annehmbare, nicht-toxische Salze mit Basen zugefügt sein.

Unter antiviral wirksamen Verbindungen vom Amantadin-Typ versteht man in erster Linie Verbindungen der Formel

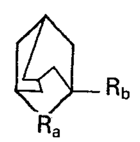 (I)

worin $R_a$ für Methylen oder Aethylen steht, und $R_b$ Amino, 1-Amino-niederalkyl, wie 1-Aminoäthyl, oder gegebenenfalls z.B. durch Diniederalkylamino, wie Dimethylamino, substituiertes Niederalkoxymethylcarbonylamino, z.B. Aethoxymethylcarbonylamino oder 2-Dimethylaminoäthoxymethylcarbonylamino steht, oder worin $R_b$ Wasserstoff bedeutet und $R_a$ einen gegebenenfalls N-substituierten, wie N-Niederalkyl, z.B. N-Methyl enthaltenden Azacycloalkylidenrest, z.B. 3-Methyl-3-azacyclopentyliden (1-Methyl-3-pyrrolidinyliden) darstellt, oder pharmazeutisch verwendbare, nicht-toxische Säureadditionssalze davon, und in erster Linie 1-Amino-adamantan (Amantadin) oder 1-(1-Amino-äthyl)-adamantan (Rimantadin), ferner 1'-Methyl-spiro[adamantan-2,3'-pyrrolidin], 1-(Aethoxymethylcarbonylamino)-adamantan oder 1-(2-Dimethylaminoäthoxy-methylcarbonylamino)-adamantan (Tromantadin), oder pharmazeutisch annehmbare, nicht-toxische Salze davon.

Geeignete Salze von obgenannten wie auch von weiter unten genannten Verbindungen mit basi-

schen Eigenschaften, sind pharmazeutisch annehmbare, nicht-toxische Säureadditionssalze mit anorganischen Säuren, z.B. Chlorwasserstoff-, Bromwasserstoff-, Salpeter-, Schwefel- oder Phosphorsäuren, oder mit organischen Säuren, wie organischen Carbonsäuren, z.B. Essigsäure, Propionsäure, Glykolsäure, Bernsteinsäure, Maleinsäure, Hydroxymaleinsäure, Methylmaleinsäure, Fumarsäure, Apfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxy-benzoesäure, Embonsäure, Nicotinsäure oder Isonicotinsäure, oder organischen Sulfonsäuren, z.B. Methansulfonsäure, Aethansulfonsäure, 2-Hydroxy-äthansulfonsäure, Aethan-1,2-disulfonsäure, Benzolsulfonsäure, 4-Methyl-benzolsulfonsäure oder Naphthalin-2-sulfonsäure.

Die oben oder weiter unten genannten Verbindungen, die Asymmetriezentren enthalten, können in Form von Racematen oder von optisch aktiven Antipoden, oder bei Vorliegen von Diastereomerie gegebenenfalls auch in Form von Racematgemischen verwendet werden.

Die in den erfindungsgemässen Präparaten enthaltenen Dosen der antiviral wirksamen Verbindungen vom Amantadin-Typ hängen in erster Linie von deren Wirkungsstärke ab.

Üblicherweise wendet man die für die Einzelkomponente als wirksam festgelegte Dosis an; doch kann man, wie insbesondere auch von Salicylamid oder Propyphenazon, auch eine gegenüber dieser verminderte, aber vorzugsweise nicht weniger als ein Viertel davon betragende Dosis verwenden.

Die antiviral wirksame Verbindung vom Amantadintyp, wie diejenigen der Formel I, insbesondere das 1-Aminoadamantan oder ein Salz davon, z.B. das Hydrochlorid, wird im pharmakologischen Versuch z.B. in Dosen von etwa 5 mg/kg bis etwa 30 mg/kg, und in der Praxis bei Warmblütern von etwa 60–70 kg Körpergewicht z.B. in Einzeldosen von 25 mg bis 100 mg oder in Tagesdosen von 50 bis 200 mg angewendet. Die einzeln und pro Tag verabreichten Dosen betragen ca. ¼ bis ¹⁄₁, vorzugsweise ca. ½ der üblicherweise therapeutisch verabreichten Dosen.

Die Einzeldosis von Salicylamid beträgt im pharmakologischen Versuch etwa 1 bis etwa 25 mg/kg, und in der Praxis, d.h. beim Warmblüter von etwa 60–70 kg Körpergewicht von 25 bis 250 mg, bei Tagesdosen von 50 bis 1500 mg, insbesondere von 50 bis 1000 mg, und für Propyphenazon im pharmakologischen Versuch von etwa 1 mg/kg bis etwa 10 mg/kg und in der Praxis von 25 bis 250 mg, bei Tagesdosen von 50 bis 500 mg.

Als gegebenenfalls beizufügende antiallergische Wirkstoffe eignen sich insbesondere Verbindungen mit Histamin-antagonistischer Wirkung, beispielsweise solche vom Imidazolin-Typ, wie 2-[(N-Benzyl-N-phenylamino)-methyl]-2-imidazolin (Antazolin), und vom Phenothiazin-Typ, wie 10-[2-(Dimethylamino)-propyl]-phenothiazin (Promethazin), oder pharmazeutisch annehmbare, nicht-toxische Säureadditionssalze von solchen Verbindungen.

Bei den gegebenenfalls beizufügenden Stoffen mit blockierender Wirkung auf α-adrenergische Rezeptoren handelt es sich beispielsweise um Verbindungen vom Imidazolin-Typ, wie 2- [N-(m-Hydroxyphenyl)-N-(p-tolyl)-amino]-methyl]-2-imidazolin (Phentolamin) oder 2-Benzyl-2-imidazolin (Tolazolin). Die antiallergischen wie auch die α-blockierenden Wirkstoffe werden in den für die einzelnen Verbindungen festgelegten oder vorzugsweise verminderten Einzeldosen beigefügt. Im allgemeinen wird ca. ¼ bis ½ der üblichen Einzeldosis beigefügt, mit einzelnen besonders geeigneten Wirkstoffen kann aber schon mit wesentlich niedrigeren Dosen eine ausreichende Wirkung erzielt werden. Besonders deutlich und zugleich schon in stark verminderten Dosen, d.h. im pharmakologischen Versuch in Dosierungen ab 0,001 bis 0,01 mg/kg per os und in der Praxis, d.h. bei Verabreichung an Warmblüter von ca. 60–70 kg Gewicht, ab ca. 0,5 bis 1 mg per os, d.h. mit ca. ¹⁄₂₀ bis ¹⁄₁₀ der üblicherweise parenteral verabreichten Dosis, feststellbar ist die Wirkung des α-Rezeptoren-blockierenden Phentolamin. Ebenfalls in Dosierungen ab 0,001 bis 0,01 mg/kg ist im pharmakologischen Versuch der wirkungssteigernde Effekt des zur gleichen Wirkstoffgruppe gehörenden Tolazolin feststellbar; dieses kann in der Praxis ebenfalls in der gegenüber der Einzeldosis von 25 mg für Warmblüter von ca. 50–70 kg Gewicht wesentlich verminderten Dosierung von ca. 0,5 bis 5 mg beigefügt werden.

Die erfindungsgemässen pharmazeutischen Präparate enthalten ausser den pharmakologischen Wirkstoffen üblicherweise geeignete Träger- und Hilfsstoffe, welche eine Verarbeitung der Wirkstoffe in die pharmazeutisch verwendbaren Präparate erleichtern. Vorzugsweise enthalten die Präparate, in erster Linie oral verabreichbare Präparate, welche für die bevorzugte Applikationsart in Frage kommen, wie Tabletten, Dragées und Kapseln, ferner auch rektal verabreichbare Präparate, wie Suppositorien, sowie geeignete Lösungen von etwa 10% bis 100% an Wirkstoffen zusammen mit dem Trägermaterial, wobei oral verabreichbare Präparate vorzugsweise etwa 20% bis 100%, insbesondere etwa 50% bis etwa 90%, und rektal verabreichbare Präparate etwa 10% bis 90% an Wirkstoffen enthalten.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man die Wirkstoffe mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethyl-cellulosephthalat, verwendet. Durch Einarbeiten eines oder mehrerer Wirkstoffe in ein geeignetes Trägermaterial, das eine langsame Abgabe des oder der Wirkstoffe bewirkt, kann die Wirkungsdauer einer oder mehrerer an sich kurz wirksamer Komponenten verlängert werden. Den Tabletten oder Dragée-Überzügen können Farbstoffe oder Pigmente, z.B. zur Identifizierung oder zur Kennzeichnung verschiedener Kombinationen von Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können die Wirkstoffe in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln sind die Wirkstoffe vorzugsweise in geeigneten Flüssigkeiten, wie fetten Ölen, Paraffinöl oder flüssigen Polyäthylenglykolen, gelöst oder suspendiert, wobei ebenfalls Stabilisatoren, z.B. Lecithine zugefügt sein können.

Als rektal anwendbare pharmazeutische Präparate kommen, z.B. Suppositorien in Betracht, welche aus einer Kombination der Wirkstoffe mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die aus einer Kombination der Wirkstoffe mit einer Grundmasse bestehen; als Grundmassenstoffe kommen z.B. flüssige Triglyceride, Polyäthylenglykole oder Paraffinkohlenwasserstoffe in Frage.

Zur oralen Verabreichung kommen im weiteren auch nicht-einzeldosierte Präparate, insbesondere in üblicher Weise bereitete Sirupe mit einem Gehalt von etwa 1% bis etwa 12% an Wirkstoffen in Betracht. Beispielsweise werden Sirupe, die 1-Amino-adamantan oder ein pharmazeutisch annehmbares, nicht-toxisches Säureadditionssalz desselben enthalten, an Warmblüter von etwa 8 bis etwa 25kg Körpergewicht zweimal täglich in einer Menge verabreicht, die etwa 3mg/kg Körpergewicht entspricht.

Die nachfolgenden Beispiele illustrieren die oben beschriebene Erfindung; sie sollen jedoch diese in ihrem Umfang in keiner Weise einschränken.

Beispiel 1

Je 100mg Salicylamid, vermischt mit 75mg 1-Aminoadamantan-hydrochlorid und 45mg Reisstärke, werden in Hartgelatinekapseln abgefüllt. Zur Behandlung von Viruserkrankungen wird an Warmblüter von etwa 50 bis etwa 70kg Körpergewicht zweimal täglich eine Kapsel verabreicht.

Beispiel 2

Je 100mg kristallisiertes 2,3-Dimethyl-4-isopropyl-1-phenyl-3-pyrazolin-5-on, vermischt mit 75g 1-Aminoadamantan-hydrochlorid und 45mg Reisstärke, werden in Hartgelatine-Kapseln abgefüllt, die in gleicher Weise verabreicht werden wie die Kapseln von Beispiel 1.

Beispiel 3

Je 50mg Salicylamid, vermischt mit 50mg 1-Aminoadamantan-hydrochlorid und 60mg Reisstärke, werden in Hartgelatine-Kapseln abgefüllt. Zur Behandlung von Viruserkrankungen wird an Warmblüter von etwa 50 bis etwa 70kg Körpergewicht zwei bis dreimal pro Tag eine Kapsel verabreicht.

Beispiel 4

Je 25mg kristallisiertes 2,3-Dimethyl-4-isopropyl-1-phenyl-3-pyrazolin-5-on, vermischt mit 25mg 1-Aminoadamantan-hydrochlorid und 30mg Reisstärke, werden in Hartgelatine-Kapseln abgefüllt. Zwei- bis dreimal täglich wird je eine dieser Kapseln an Warmblüter von etwa 25 bis 50kg Körpergewicht verabreicht. Man kann auch an Warmblüter von höherem Körpergewicht eine entsprechend diesem Gewicht vermehrte Zahl Kapseln mehrmals pro Tag verabreichen.

Beispiel 5

Je 25mg Salicylamid, vermischt mit 25mg 1-Aminoadamantan-hydrochlorid und 30mg Reisstärke, werden in Hartgelatine-Kapseln abgefüllt, die in gleicher Weise verabreicht werden wie die Kapseln von Beispiel 4.

Beispiel 6

Je 50mg kristallisiertes 2,3-Dimethyl-4-isopropyl-1-phenyl-3-pyrazolin-5-on, vermischt mit 50mg 1-Amino-adamantan-hydrochlorid, 1,0mg 2-[N-(m-Hydroxyphenyl)-N-(p-tolyl)-amino]-methyl]-2-imidazolin-methansulfonat und 60mg Reisstärke, werden in Hartgelatine-Kapseln abgefüllt, die in gleicher Weise verabreicht werden wie die Kapseln von Beispiel 3.

**Patentansprüche**

1. Synergistisch wirksames, pharmazeutisches Präparat zur Behandlung von Virusinfektionen enthaltend eine antiviral wirksame Verbindung vom Amantadin-Typ oder ein pharmazeutisch annehmbares, nicht-toxisches Säureadditionssalz einer solchen in Kombination mit Salicylamid oder Propyphenazon.

2. Pharmazeutisches Präparat nach Anspruch 1, enthaltend zusätzlich eine antiallergisch wirksame Verbindung.

3. Pharmazeutisches Präparat nach Anspruch 1, enthaltend zusätzlich eine auf α-adrenergische Rezeptoren blockierend wirkende Verbindung.

4. Pharmazeutisches Präparat nach Anspruch 1, enthaltend als antiviral wirksame Verbindung vom Amantadin-Typ eine solche der Formel

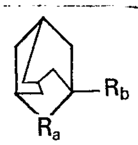

(I)

worin $R_a$ für Methylen oder Äthylen steht, und $R_b$ für Amino-1-Amino-niederalkyl in Form von 1-Aminoäthyl, oder gegebenenfalls substituiertes Niederalkoxymethylcarbonylamino in Form von Äthoxymethylcarbonylamino, oder 2-Dimethylaminoäthoxymethylcarbonylamino steht, oder worin $R_b$ Wasserstoff bedeutet und $R_a$ einen gegebenenfalls N-substituierten Azacycloalkylidenrest darstellt, oder ein pharmazeutisch annehmbares, nicht-toxisches Säureadditionssalz davon.

5. Pharmazeutisches Präparat nach Anspruch 1, enthaltend als antiviral wirksame Verbindung 1-Amino-adamantan oder ein pharmazeutisch annehmbares, nicht-toxisches Säureadditionssalz davon.

6. Pharmazeutisches Präparat nach Anspruch 1, enthaltend als zweite Wirkstoffkomponente Salicylamid.

7. Pharmazeutisches Präparat nach Anspruch 1, enthaltend als zweite Wirkstoffkomponente Propyphenazon.

8. Pharmazeutisches Präparat nach Anspruch 1, enthaltend eine jeweilig kombinierte Einzeldosis von 25 bis 100 mg 1-Aminoadamantan oder einem pharmazeutisch annehmbaren, nicht-toxischen Säureadditionssalz davon, und 25 bis 250 mg Salicylamid.

9. Pharmazeutisches Präparat nach Anspruch 1, enthaltend eine jeweilig kombinierte Einzeldosis von 25 bis 100 mg 1-Aminoadamantan oder einem pharmazeutisch annehmbaren, nicht-toxischen Säureadditionssalz davon, und 25 bis 250 mg Propyphenazon.

10. Pharmazeutische Präparate nach Ansprüchen 1 bis 9, gegebenenfalls mit Trägerstoffen und Hilfsstoffen, in einer für die orale Verabreichung geeigneten Applikationsform.

11. Pharmazeutisches Präparat nach Ansprüchen 1 bis 9 mit Trägerstoffen und Hilfsstoffen in einer für die rektale Verabreichung geeigneten Applikationsform.

**Claims**

1. A pharmaceutical preparation having synergistic action for the treatment of virus infections, said preparation containing an antiviral compound of the amantadine type or a pharmaceutical acceptable non-toxic acid addition salt thereof, in combination with salicylamide or propyphenazone.

2. A pharmaceutical preparation according to claim 1, which additionally contains a compound having antiallergic action.

3. A pharmaceutical preparation according to claim 1, which additionally contains a compound which blocks α-adrenergic receptors.

4. A pharmaceutical preparation according to claim 1, wherein the antiviral compound of the amantadine type is a compound of the formula

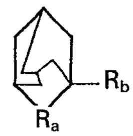

(I)

wherein $R_a$ is methylene or ethylene and $R_b$ is amino, 1-aminolower alkyl, in terms of 1-aminoethyl, or unsubstituted or substituted lower alkoxymethylcarbonylamino, in terms of ethoxymethylcarbonylamino or 2-dimethylaminoethoxymethylcarbonylamino, or wherein $R_b$ is hydrogen and $R_a$ is an unsubstituted or a N-substituted azacycloalkylidene radical, or a pharmaceutically acceptable non-toxic acid addition salt thereof.

5. A pharmaceutical preparation according to claim 1, wherein the antiviral compound is 1-amino-adamantane or a pharmaceutically acceptable non-toxic acid addition salt thereof.

6. A pharmaceutical preparation according to claim 1, which contains salicylamide as second active component.

7. A pharmaceutical preparation according to claim 1, which contains propyphenazone as second active component.

8. A pharmaceutical preparation according to claim 1, which contains a single dose combined in each case of 25 to 100 mg of 1-aminoadamantane or a pharmaceutically acceptable non-toxic acid addition salt thereof, and 25 to 250 mg of salicylamide.

9. A pharmaceutical preparation according to claim 1, which contains a single dose combined in each case of 25 to 100 mg of 1-aminoadamantane or a pharmaceutically acceptable non-toxic acid addition salt thereof, and 25 to 250 mg of propyphenazone.

10. A pharmaceutical preparation according to any one of claims 1 to 9, optionally together with carriers and adjuncts, in a formulation suitable for oral administration.

11. A pharmaceutical preparation according to any one of claims 1 to 9, optionally together with carriers and adjuncts, in a formulation suitable for rectal administration.

**Revendications**

1. Préparation pharmaceutique à action synergique pour le traitement des infections virales contenant un composé à action anti-virale du type de l'amantadine ou un sel d'addition d'acide non toxique pharmaceutiquement acceptable d'un tel composé en combinaison avec le salicylamide ou la propyphénazone.

2. Préparation pharmaceutique selon la revendication 1, contenant en outre un composé à action anti-allergique.

3. Préparation pharmaceutique selon la revendication 1, contenant en outre un composé ayant une action bloquant les récepteurs α-adrénergiques.

4. Préparation pharmaceutique selon la revendication 1, contenant comme composé à action antivirale du type de l'amantadine un composé de formule

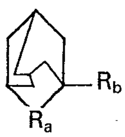 (I)

où $R_a$ représente un méthylène ou en éthylène, et $R_b$ représente un amino, un 1-amino-alcoyle inférieur, savoir un 1-aminoéthyle, ou un alcoxy inférieur-méthylcarbonylamino éventuellement substitué, savoir un éthoxymethylcarbonylamino ou un 2-dimethylaminoéthoxymethylcarbonylamino, ou bien où $R_b$ représente un hydrogène et $R_a$ représente un radical azacycloalcoylidene éventuellement N-substitué, ou un de ses sels d'addition d'acides non toxique, pharmaceutiquement acceptable.

5. Préparation pharmaceutique selon la revendication 1, contenant comme composé à action antivirale le 1-amino-adamantane ou un de ses sels d'addition d'acides non toxique pharmaceutiquement acceptable.

6. Préparation pharmaceutique selon la revendication 1, contenant comme second composant actif le salicylamide.

7. Préparation pharmaceutique selon la revendication 1, contenant comme second composant actif la prophénazone.

8. Préparation pharmaceutique selon la revendication 1, contenant une dose unique chaque fois combinée de 25 à 100 mg de 1-aminoadamantane ou d'un de ses sels d'addition d'acides non toxique pharmaceutiquement acceptable, et de 25 à 250 mg de salicylamide.

9. Préparation pharmaceutique selon la revendication 1, contenant une dose unique chaque fois combinée de 25 à 100 mg de 1-amino-adamantane ou d'un de ses sels d'addition d'acide non toxique pharmaceutiquement acceptable, et de 25 à 250 mg de propyphénazone.

10. Préparations pharmaceutiques selon les revendications 1 à 9, éventuellement avec des supports ou additifs, sous une forme d'application appropriée à l'administration orale.

11. Préparations pharmaceutiques selon les revendications 1 à 9 avec des supports et additifs sous une forme d'application appropriée à l'administration rectale.